# EUROPEAN PATENT APPLICATION

(11) **EP 0 646 357 A1**
(43) Date of publication of application: **05.04.1995**
(21) Application number: 94307127.4
(22) Date of filing: 29.09.1994
(51) Int. Cl.: A61B 17/28

(54) **Surgical instrument having improved manipulating means**

(30) Priority: 30.09.1993 US 130065
(71) Applicant: ETHICON, INC., Somerville, NJ 08876 (US)
(72) Inventor: Butterfield, Eric J., Middletown, Ohio 45044 (US)
(74) Representative: Fisher, Adrian John

(57) **Abstract**

A surgical instrument having a business end manipulated by a member made from a cobalt-sterilizable, nontoxic, liquid-crystal, polyester-polyarylate polymer.

## Description

### Background of the Invention

Over the past years, instruments used in surgery have continued to increase in complexity. Whereas instruments such as forceps, retractors and graspers have been used for some time, newer instruments such as clip appliers, staplers and the like are continuing to gain in acceptance. As more and more instruments are developed for surgery, they continue to become more complex. Examples of such complex instruments are linear staplers, circular staplers, ligating instruments, ligators and dividers. With the advent of endoscopic surgery, these types of instruments have become smaller in size.

Many instruments have been developed to perform some type of surgical function, such as ligating, stapling, cutting or otherwise manipulating tissue. At present, many of these instruments are being improved to make them easier to use. One example of this is to make the business end of the instrument rotatable so that it can be used at various positions within the surgical. site. Another example is to make the distal end or business end of the instrument articulatable; i.e., provide angular motion to the business end so that it may be easily fitted to various configurations of the human anatomy. In the past, most of these complex instruments have been actuated or manipulated through various types of mechanical linkages. While these linkages have gained some acceptance, there are problems of size and space limitation accompanying such linkages, especially when used in endoscopic instruments. As can be appreciated, many of the size and space limitations may be overcome by using cables or pulleys to accomplish similar mechanical manipulation. Such cables require less space and are able to follow tortuous paths while still transmitting the desired forces. Also, the cable is generally a single piece and, hence, there are less tolerance problems. All of these fasteners reduce the problems encountered in the design of surgical instruments, especially endoscopic surgical instruments.

While it is known that cables have certain advantages when used as the manipulating member in a surgical instrument, many of the cables still have serious problems. Steel cables, while providing excellent strength when used as a manipulating member, if used in any type of tortuous path where they have to pass around a relatively tight bend, will take a "set" which reduces the reliability of the instrument.

It is an object of the present invention to provide a manipulative member in a surgical instrument that is cobalt sterilizable. It is a further object of the present invention to provide a manipulative member that has excellent strength and can follow tortuous paths in relatively limited space. It is a further object of the present invention to provide a manipulative member for use in surgical instruments that has high tensile strength so that considerable forces can be applied utilizing the member.

### Summary of the Invention

What I have discovered is a new and improved surgical instrument. The instrument has a distal end used to carry out a step in a surgical procedure such as applying a clip, setting a staple, cutting tissue, grasping tissue or various combinations of such steps. The instrument has a proximal end which incudes the actuation means for manipulating and controlling the distal end or business end of the instrument. The distal end and proximal end are connected by a shaft, housing, or other suitable structure. In the preferred instruments of the present invention, they are connected by a relatively small diameter, elongated shaft so that the instrument may be used in endoscopic surgery.

The instruments of the present invention include a member which extends through the shaft from the proximal end to the distal end of the instrument. This member allows for the manipulation of the distal end of the instrument. At least a portion of this manipulating member is a cobalt sterilizable, nontoxic, liquid-crystal, polyester-polyarylate polymer. The liquid crystal polymer has high tensile properties and low elongation. Preferably, the manipulating member is in the form of a "cord" or "cable" or other flexible strand-like member made from multifilament fiber. The cord may be a single strand of the multifilament fiber or it may be multiple strands of the multifilament fiber. Examples of such fibers useful in cords or cables in performance of the invention are fibers made of materials such as Vectran™, Vectra™, or Spectra™, all manufactured by Hoechst-Celanese, Bridgewater, New Jersey. In addition, it is believed that Kevlar™, manufactured by E.I. Dupont de Nemours, Wilmington, Delaware, will also perform adequately. It is intended by the scope of this invention to include all polymer cords with high tensile strength (greater than at least 10 pounds) and low elongation (less than 10%), which are nontoxic and sterilizable. Exemplary of such polyesters/polyarylates are liquid crystal thermoplastic copolymers and thermoplastic monopolymers and copolymers in general. Also, such polymers are well described in U.S. Patent Nos. 4,183,895; 4,450,737; 4,799,985, all incorporated herein by reference.

### Brief Description of the Drawings

In the accompanying drawings that form part of the specification, like numerals are employed to designate like parts throughout the same;
FIGURE 1 is a simplified, exploded, perspective view of the distal end of a portion of an instrument of the present invention;
FIGURE 2 is a simplified, schematic representation of the proximal portion of the instrument showing the handle and a lever for operating the distal end;
FIGURE 3 is a fragmentary, top plan view of the distal end jaw assembly illustrated in FIGURE 1, but FIGURE 3 shows the jaw assembly in an open position with portions of the upper or second jaw cut away to illustrate interior detail;
FIGURE 4 is a fragmentary, cross-sectional view taken generally along the plane 4-4 in FIGURE 3;
FIGURE 5 is a view similar to FIGURE 4, but FIGURE 4 shows a partially closed condition of the jaw assembly;
FIGURE 6 is a view similar to FIGURE 5 but shows a completely closed condition of the jaw assembly;
FIGURE 7 is a simplified, perspective view of another embodiment of an instrument of the present invention;
FIGURE 8 is a perspective view of the manipulating mechanism used in the instrument depicted in FIGURE 7;
FIGURE 9 is an enlarged schematic sectional view of the articulation pin depicted in FIGURE 8; and
FIGURE 10 is an enlarged schematic sectional view of the pivot pin depicted in FIGURE 8.

### Detailed Description of the Drawings

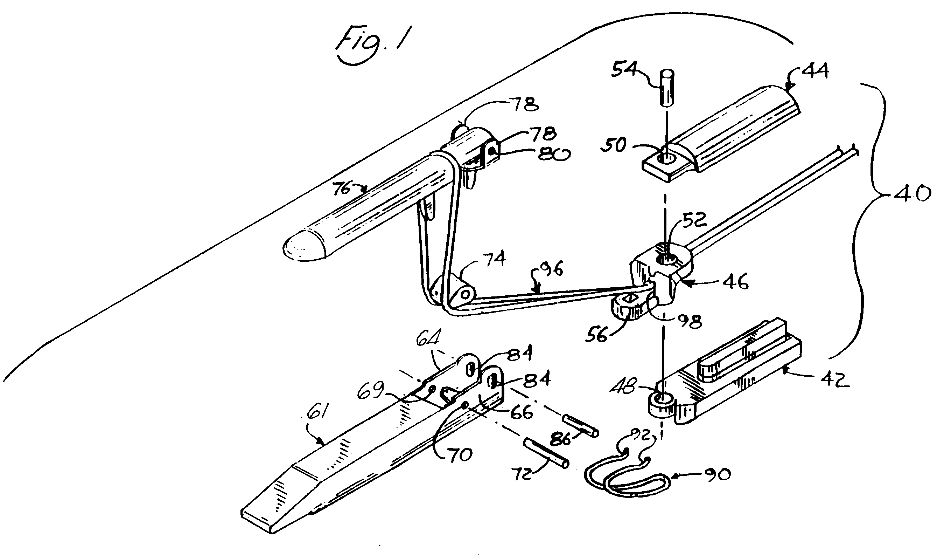

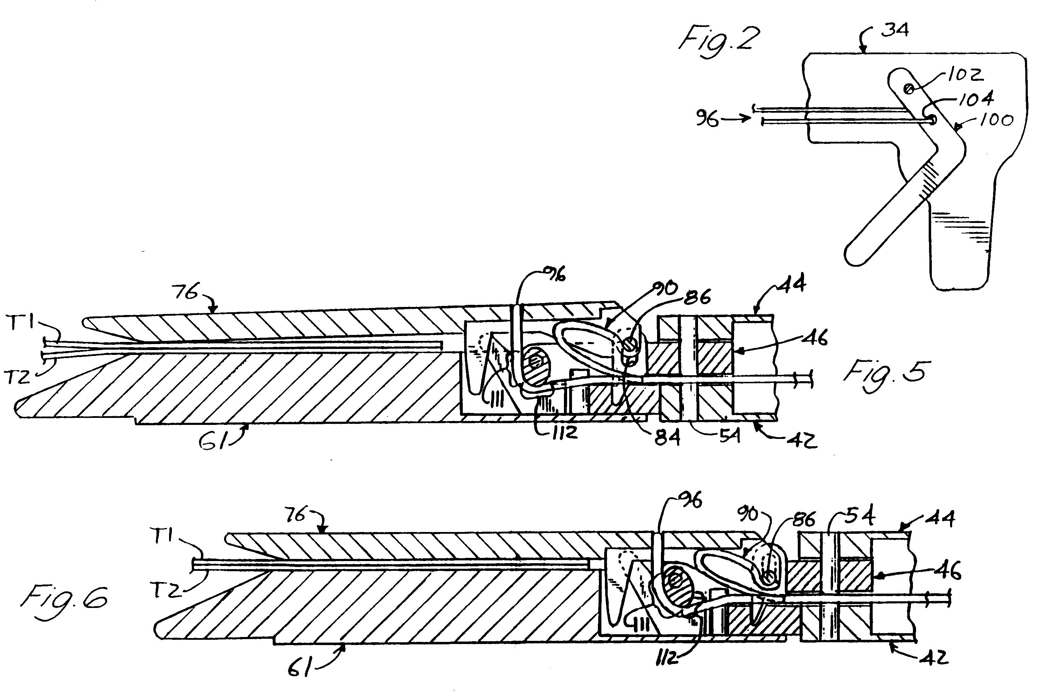

FIGURES 1-6 schematically illustrate one form of surgical instrument which embodies the present invention. In the embodiment depicted, the cobalt sterilizable, nontoxic, liquid-crystal, polyester-polyarylate, polymer manipulating member is a cord of multifilament fiber and is used to control the movement of a pair of jaws disposed at the distal or business end of the instrument. The polyester-polyarylate fiber has high tensile properties (i.e., strength and modulus) in order to transmit the considerable forces required to manipulate the business end of the instrument. The high tensile properties also reduce the possibility of stretching, deformation and even breakage of the manipulating member during operation of the instrument. Furthermore, the manipulating member has low elongation and no measurable creep up to fifty percent of its breaking strength. The jaws of the instrument may be constructed to apply staples to tissue, to apply a ligating clip to a vessel, to grasp tissue or to carry out other functions used in surgical procedures.

FIGURE 1 depicts a jaw assembly that may be mounted to a proximal portion of an open surgery or endoscopic instrument, and the proximal portion may typically be a support housing 34 (FIGURE 2). This part of the instrument is grasped by the surgeon. In an endoscopic instrument, the proximal part of the housing remains outside of the patient while the rest of the instrument is inserted through the trocar cannula (not illustrated) and into the body cavity.

The jaw assembly can be pivotally mounted to the instrument, and to this end the instrument includes a mounting assembly 40 (FIGURE 1) comprising a bottom bracket 42, a top bracket 44, and a support member 46. The lower bracket defines a bore 48, the upper bracket 44 defines a bore 50, and the support member 46 defines a bore 52. The components are assembled so that the bores 48, 52, and 50 are aligned to receive a hinge, pin or pivot pin 54 (FIGURES 1 and 4). The proximal portions of the lower bracket 42 and upper bracket 44 are adapted to be mounted to the housing 34 by suitable means (not illustrated).

The support member 46 defines a cantilevered tongue 56 on which the jaw assembly is mounted. To this end, the jaw assembly includes a first or lower jaw 61 having a proximal portion in the form of a channel defined by a first side wall 64, a second side wall 66 and a bottom wall or floor 68 (FIGURE 4). The projecting tongue 56 of the support member 46 is received within the proximal portion of the first jaw 61 as shown in FIGURE 4 and is retained therein by suitable means (e.g., threaded fasteners, press fit, adhesive, welding or brazing, and the like (not illustrated). The particular means by which the jaw assembly is attached to the tongue 56 or to any other portion of an instrument as may be desired, forms no part of the present invention.

The first jaw side wall 64 defines an aperture 69, and the first jaw side wall 66 defines an aperture 70. An oval lever arm 74 is disposed on a pin 72 which is mounted in the apertures 69 and 70 in the first jaw 61.

The jaw assembly includes an upper or second jaw 76 which has a proximal end portion defining a pair of bosses or ears 78. Each boss 78 defines a bore 80. The proximal portion of the second jaw 76 is received between the first jaw side walls 64 and 66. Each side wall 64 and 66 defines a vertically oriented, elongated slot 84. A shaft 86 is mounted through the slots 84 and through the second jaw bores 80 so as to retain the second jaw 76 mounted on the first jaw 61.

The length of each slot 84 is greater than the diameter of the shaft 86. The shaft 86 is thus free to translate vertically within the slots 84. This accommodates movement of the proximal portion of the second jaw 76 toward and away from the first jaw 61. Further, the shaft 86 may be characterized as defining a pivot axis about which the second jaw 76 can pivot. This pivot axis is, of course, not fixed and can translate relative to the first jaw 61 as the shaft 86 moves along the slots 84.

In the embodiment illustrated, a spring 90 is mounted within the jaw assembly as illustrated in FIGURES 1 and 4. The spring 90 has a generally U-shaped configuration with the legs of the U bent over to define cradle portions 92 which engage the shaft 86 so as to bias the shaft, and hence, the proximal end of the second jaw 76, upwardly away from the first jaw 61.

In the embodiment depicted in FIGURES 1-6, the manipulating member is a cord 96. The cord is a single strand of a cobalt sterilizable, nontoxic, liquid-crystal, polyester-polyarylate polymer in the form of a multi-filament fiber. If desired, the cord could be multiple strands of the multi-filament fiber polymer. Such multiple strands could be braided or formed into a cable if desired. Furthermore, the cord of the multi-filament polyester-polyarylate fiber could be combined with strands or ends of the materials such as a steel wire or high performance fibers such as animal fibers or polyethylene fibers or the like.

The cord 96 defines a generally U-shaped configuration around the second jaw 76 and extends from the second jaw 76 around the lever 74. The cord 96 extends from the lever 74 beyond the proximal end of the jaw assembly where it may be pulled by a suitable means. In the embodiment illustrated in FIGURES 1-6, the instrument support member 46 defines an aperture 98 through which the cord 96 can extend proximally from the jaws 61 and 76. The cord 96 preferably extends to a proximal portion of the instrument, such as to the proximal end of the housing 34 (FIGURE 2) which can be pulled by the surgeon.

A preferred system for pulling the cord 96 is illustrated in FIGURE 2 and comprises an L-shaped lever 100 pivotally mounted with a pin 102 to the housing 34. The loop of cord 96 extends through an aperture 104 in the operating lever 100. Because the spring 90 normally biases the second jaw 76 to the open position, the cord 96 is normally pulled distally so as to pull the operating lever 100 to a forward position against some suitable stop (not illustrated) in the housing 34. The surgeon can hold the proximal end of the housing 34 and squeeze the lever 100 rearwardly to pull the cord 96 proximally.

The distal end of the loop defined by the cord 96 is engaged with the second jaw 76. To this end, the outer surface of the second jaw 76 defines an arcuate groove 108 (FIGURE 4) in which the cord 96 is seated. The cord 96 extends down both sides of the second jaw 76 into the channel section of the first jaw 61. The cord 96 is trained around, and engaged with the exterior surface of the oval lever 74.

The lever 74 need not necessarily be oval but should have a first surface 111 and a second surface 112 (FIGURE 4) wherein the distance between the lever pivot axis and any part of the first surface 111 is less than the distance between lever pivot axis and any part of the second surface 112. Preferably, the lever 74 is initially installed and engaged with the cord 96 such that the lever 74 has the orientation shown in Figure 4 when the second jaw 76 is fully open. In particular, in this condition, the longest portion of the lever 74 (e.g., the major axis of an oval-shaped lever) is oriented generally parallel to the lower jaw 61 and is generally parallel to the length of the instrument along which the proximal portion of the loop of cord 96 is pulled. The cord 96 extending between the lever 74 and the second jaw 76 defines an angle of almost 90° with respect to the length of cord 96 extending proximally from the lever 74.

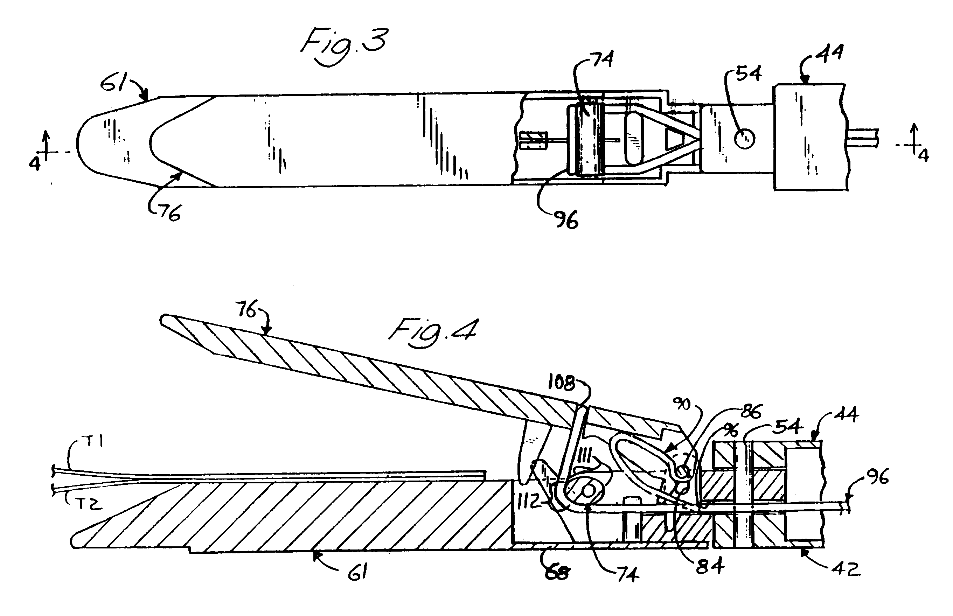

When the cord 96 is pulled proximally, the length of cord along the first surface 111 applies tension through a relatively short lever arm while the portion of the cord along the second surface 112 applies tension through a relatively long lever arm. As the cable 96 is pulled proximally, the lever 74 pivots (counterclockwise as viewed in FIGURE 4). As the lever pivots to the intermediate position illustrated in FIGURE 5, the length of cord 96 between the lever 74 and second jaw 76 is subjected to a relatively long travel at a relatively lower force. As the cord 96 continues to be pulled proximally from the orientation illustrated in FIGURE 5 to the orientation illustrated in FIGURE 6, the portion of the cord 96 engaged with the first surface 112 applies tension through a relatively long lever arm while the portion of the cord extending from the lever arm 74 to the second jaw 76 is tensioned through a relatively short lever arm. Thus, the travel of the cord 96 adjacent the second surface 112 is translated into a shorter travel and is subjected to a higher force.

The effect of the operation of the jaw assembly is to initially move the second jaw 76 through a relatively large percentage of the distance toward the closed position at relatively low force and to subsequently pivot the second jaw 76 through a short, remaining arc to the fully closed position at a relatively high force. For example, if the lever 74 is elliptical, and if the length of the major axis is equal to twice the length of the minor axis, then the second jaw 76 will be initially pulled toward the fully closed position with a force equal to 1/2 of the input pulling force and will pull through an arc (as measured at the radius where the cord 96 engages the top of the second jaw 76) having a length equal to twice the initial pulling stroke length. Subsequently, when the lever 76 has pivoted about 90° (to the position illustrated in Figure 5), the second jaw 76 will apply a clamping force equal to twice the pulling force as the second jaw 76 moves through an arc length equal to 1/2 of the final pull stroke length.

With reference to FIGURES 4 and 5, it will be appreciated that during an initial pivoting movement of the second jaw 76, the proximal end of the second jaw 76 remains substantially elevated because the spring 90 is biasing the shaft 86 upwardly in the slots 84. Thus, as illustrated in FIGURE 5, the distal end of the second jaw 76 initially engages material (e.g., two layers of tissue T1 and T2) before the more proximal portions of the jaw 76 can engage the material. This tends to initially clamp the material between the distal ends of the two jaws and eliminate or minimize the normal tendency of material to move along conventional jaws which pivot closed about a fixed pivot axis.

After the distal portion of the second jaw 76 has been pivoted to the substantially closed position, further force exerted by the pulling cord 96 is sufficient to overcome the force of the spring 90, and the proximal end of the second jaw 76 is pulled toward the first jaw 61 until the jaw assembly is completely closed as illustrated in FIGURE 6.

The jaw assembly illustrated in FIGURES 1-6 can be used in an articulating surgical instrument. Because the jaw assembly is mounted via the first jaw 61, to the tongue 56 of the pivotal support member 46, the jaw assembly moves with the support member 46 as it pivots about the axis defined by the pin 54.

The first jaw 61 and second jaw 76 are shown as having generally solid distal end portions. It will be appreciated that one or both of the jaws may have other configurations and may include hollow portions. Indeed, one or both of the jaws may include auxiliary components for acting on the tissue that is adjacent or clamped between the jaws. For example, one or both of the jaws could incorporate sensor lines, aspiration conduits, irrigation conduits, and the like.

Further, the jaws may be adapted to apply tissue fasteners, such a clips or staples. For example, the first jaw 61 may be provided with a row or rows of staples, and the second jaw 76 may incorporate an anvil design so that both jaws can function as a linear stapler. A suitable mechanism can be provided in the staple-carrying jaw to discharge the staples through the tissue against the anvil jaw. Such a mechanism could be operated by means of an actuating member extending from the proximal end of the instrument into the staple-carrying jaw. Linear stapler designs which may be suitable for adaption and incorporation with the present invention are illustrated in U.S. Patent No. 4,610,383.

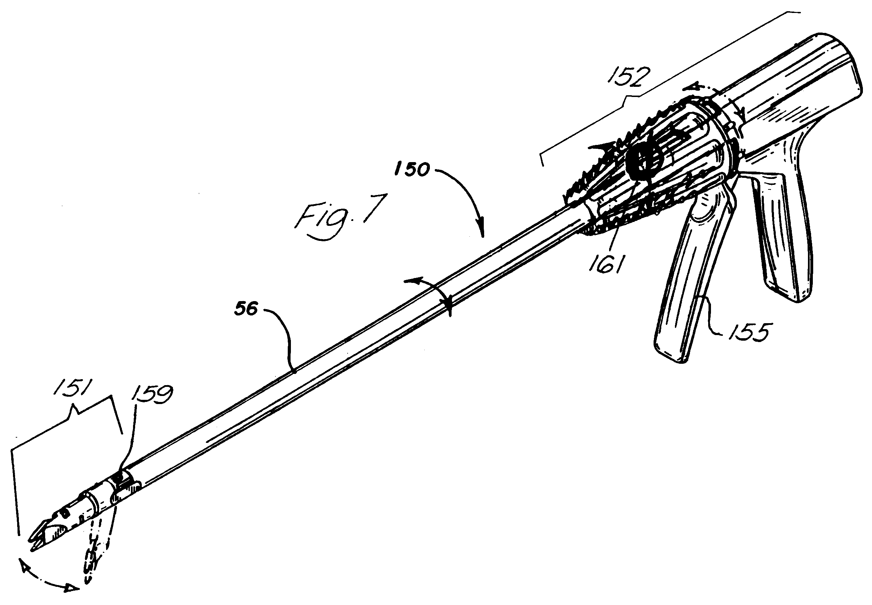

Another embodiment of the present invention is depicted in FIGURES 7 through 10. In this embodiment, the cobalt sterilizable, nontoxic, liquid-crystal, polyester-polyarylate polymer manipulating member is used to move the business end of the instrument at an angle with respect to the shaft of the instrument. In the embodiment shown, the instrument 150 comprises a distal or business end 151 for accomplishing some step in a surgical procedure such as applying ligating clips, grasping tissue, applying staples or the like.

The proximal end 152 of the instrument includes the actuating means 155 for actuating the business end of the instrument to perform the desired function. The distal and proximal ends of the instrument are connected by an elongated shaft 156. The elongated shaft allows the business end of the instrument to be appropriately placed through a trocar into the surgical environment.

Disposed in this shaft may be various types of mechanical means for transmitting the forces applied at the proximal end of the instrument to the distal end of the instrument to carry out the desired function. Such means are well known in the art and vary widely depending on the function to be performed.

The distal end of the business end of the instrument is connected to the shaft by a pivoting means 159. Such pivot means allows the distal end of the instrument to move out of alignment with the shaft; that is, to move angularly (as shown by the dotted line configuration of FIGURE 7). If desired, the shaft may also be made rotatable so that both the shaft and the business end are rotatable and the business end can move angularly out of alignment with the shaft to provide considerable freedom of movement of the business end of the instrument. This allows for considerably greater manipulation of the business end of the instrument in the surgical environment.

The pin 159 extends through the diameter of the shaft and contains a groove 160 as is more clearly shown in FIGURE 8. Disposed at the proximal end of the instrument is the articulating lever 161. The lever is disposed from a pivot pin 162 extending through the diameter of the shaft and which is in alignment with the pivot pin at the business end of the instrument. As shown in FIGURE 8, the articulating lever pivot pin also contains a groove 163. A manipulating member 164 of a cobalt sterilizable, nontoxic, liquid-crystal polyester-polyarylate polymer in the form of a cord extends in a continuous loop between the two pivot pins and rides in the grooves 160 and 163.

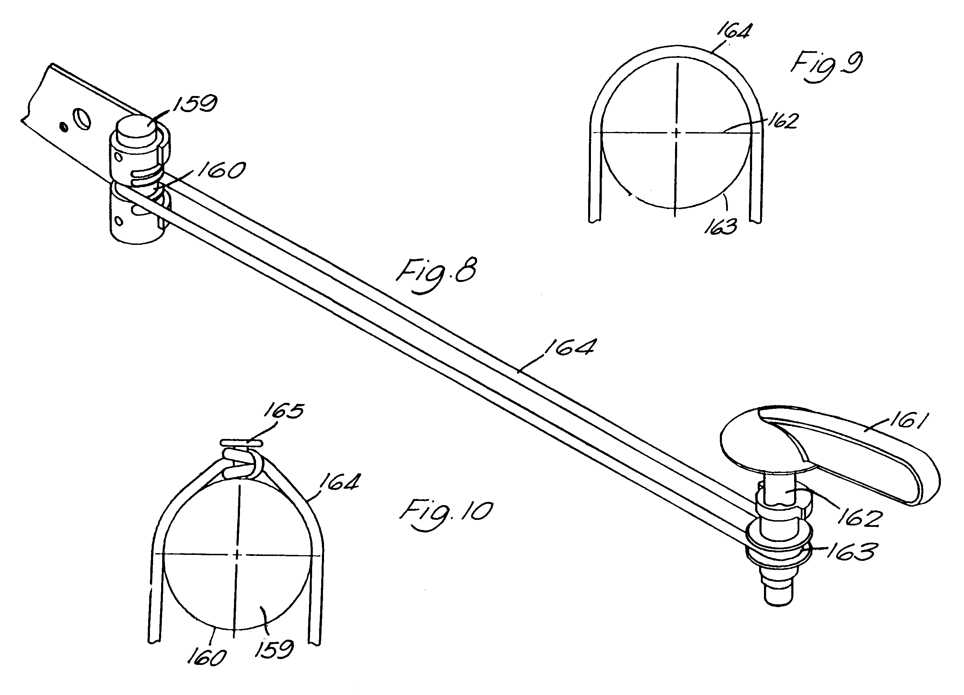

As shown in FIGURE 9, the cord 164 engages the groove 163 in the articulating lever pivot pin 162 with a friction fit. Preferably, the groove 160 in the pivot pin 159 includes a dog 165. The cord 164 is knotted or otherwise attached to the dog 165 to reduce possible slipping between the pivot pins and the cord. By turning the articulating lever 161 in either direction, the business end of the instrument is moved out of alignment with the shaft.

The particularly preferred pivot pins 160, 162 have an extension 165, 167, respectively, extending from the groove 160, 163 which allows the manipulating member to be looped or knotted about that extension 165, 167. The cord 164 may engage one or both of the pivot pins 160, 162 with a friction fit. If desired, the cord 164 may engage the pivot pins 160, 162 as depicted in FIGURES 7-10. As can be appreciated, this eliminates the possibility of slippage between the cord 164 and the pivot pins 160, 162.

The embodiments of the present invention are intended to be merely exemplary and those skilled in the art shall be able to make various variations and modifications without departing from the spirit and scope of the present invention. Furthermore, all or certain preferred embodiments of the invention described herein satisfy one or more objects and provide one or more advantages as discussed herein. It is expressly contemplated that the invention may be practiced in spirit without utilizing all of the objects indicated. Accordingly, the objects and advantages of the invention form no part thereof except as such as embodied by the scope of the following claims.

## Claims

1. A surgical instrument comprising a distal end for carrying out a step in a surgical procedure, a proximal end for actuating the manipulation of the distal end of said instrument, a structure connecting said distal end and said proximal end of said instrument, and a member extending from the actuating means in said proximal end through said structure to said distal end to allow manipulating of said distal end from said proximal end of the instrument, at least a portion of the said member being a cobalt sterilizable, nontoxic, liquid-crystal, polyester-polyarylate polymer, said polymer having high tensile properties and low elongation.

2. A surgical instrument according to Claim 1 wherein said manipulating member is a cord made of a cobalt sterilizable, nontoxic, liquid-crystal, polyester-polyarylate polymer.

3. A surgical instrument according to Claim 2 wherein said cord is a single strand of multifilament fibers.

4. A surgical instrument comprising a distal end for carrying out a step in the surgical procedure, a proximal end for manipulating the distal end of said instrument, a structure connecting said distal end and said proximal end of said instrument, said distal end being connected to said structure to allow for angular movement of said distal end with respect to said structure and a member extending from said distal end through said structure to said proximal end to provide said angular movement, at least a portion of said member being a cobalt sterilizable, nontoxic, liquid-crystal polyester-polyarylate polymer, said polymer having high tensile properties and low elongation.

5. A surgical instrument according to Claim 4 wherein the member providing angular movement is a cord made of a cobalt-sterilization, nontoxic, liquid-crystal, polyester-polyarylate polymer.

6. A surgical instrument according to Claim 5 wherein said cord is a single strand of multifilament fibers.

7. A surgical instrument comprising a distal end for carrying out a step in a surgical procedure, a proximal end for actuating the manipulation of the distal end of said instrument, a structure connecting said distal end and said proximal end of said instrument, and a member extending from the actuating means in said proximal end through said structure to said distal end to allow manipulating of said distal end from said proximal end of the instrument, at least a portion of the said member being a sterilizable, nontoxic polymer cord.

8. A surgical instrument according to Claim 7 wherein said manipulating member is a cord made of a cobalt sterilizable, nontoxic, liquid-crystal, polyester-polyarylate polymer.

9. A surgical instrument according to Claim 8 wherein said cord is a single strand of multifilament fibers.

10. A surgical instrument comprising a distal end for carrying out a step in the surgical procedure, a proximal end for manipulating the distal end of said instrument, a structure connecting said distal end and said proximal end of said instrument, said distal end being connected to said structure member to allow for angular movement of said distal end with respect to said structure and a member extending from said distal end through said structure to said proximal end to provide said angular movement, a portion of said member being a sterilizable, nontoxic polymer cord.

11. A surgical instrument according to Claim 10 wherein the member providing angular movement is a cord made of a cobalt-sterilization, nontoxic, liquid-crystal, polyester-polyarylate polymer.

12. A surgical instrument according to Claim 11 wherein said cord is a single strand of multifilament fibers.

13. A surgical instrument according to claim 10 wherein said polymer cord is a thermoplastic polymer.

14. A surgical instrument according to claim 13 wherein said thermoplastic is a polyester homopolymer or polyester copolymer.

15. A surgical instrument according to claim 13 wherein said thermoplastic is a polyester copolymer.

16. A surgical instrument according to claim 15 wherein said copolymer is a liquid crystal aromatic polyester copolymer.
